# FASCICULE DE BREVET EUROPEEN

(11) **EP 3 054 963 B1**
(45) Date de publication et mention de la délivrance du brevet: **22.08.2018**
(21) Numéro de dépôt: 14780495.9
(22) Date de dépôt: 02.10.2014
(51) Int. Cl.: A61K 36/185, A61K 8/97, A61Q 19/08, A61P 17/00

(54) **UTILISATION COSMÉTIQUE ET/OU DERMATOLOGIQUE D'UN EXTRAIT DE FEUILLES D'HAMAMELIS VIRGINIANA**
KOSMETISCHE ODER DERMATOLOGISCHE VERWENDUNG EINES HAMAMELIS-VIRGINIANA-BLÄTTER-EXTRAKTS
COSMETIC OR DERMATOLOGICAL USE OF AN EXTRACT OF HAMAMELIS VIRGINIANA LEAVES

(30) Priorité: 08.10.2013 FR 1359749
(43) Date de publication de la demande: 17.08.2016
(73) Titulaire: BASF Beauty Care Solutions France S.A.S., 69007 Lyon (FR)
(72) Inventeur: CENIZO, Valérie, 13650 Meyrargues (FR); ANDRE, Valérie, F-69420 Ampuis (FR); GAILLARD, Christelle, F-01800 Meximieux (FR)
(74) Mandataire: Mendelsohn, Isabelle M. N.
(86) Numéro de dépôt international: PCT/EP2014/071201
(87) Numéro de publication internationale: WO 2015/052082

(56) Documents cités:
- WO-A2-2007/048985
- DE-A1- 10 118 382
- FR-A1- 2 735 981
- FR-A1- 2 855 968
- FR-A1- 2 902 340
- FR-A1- 2 915 101
- FR-A1- 2 954 702
- US-A1- 2013 149 398
- Mintel Group: "Skin Spray Product Description", , 1 février 2012 (2012-02-01), XP055122674, Extrait de l'Internet: URL:http://www.gnpd.com/sinatra/recordpage /1724291/from_search/uszqZShwg3/ [extrait le 2014-06-11]

## Description

La présente invention concerne un nouvel agent cosmétique et/ou dermatologique et son utilisation cosmétique ou pharmaceutique en particulier dermatologique pour augmenter la formation des fibres de la matrice extracellulaire et pour augmenter l'élasticité de la peau et/ou des muqueuses, notamment oculaires, labiales et gingivales, et/ou du cuir chevelu.

Le vieillissement cutané en particulier le vieillissement photo-induit est un phénomène complexe impliquant plusieurs mécanismes largement étudiés tels que la production de composés réactifs ROS (Reactive oxygen species) en particulier les radicaux libres responsables de phénomènes d'oxydation (peroxydation lipidique et oxydation des protéines) et la production d'enzymes tels que les élastases et les métalloprotéinases à l'origine de la destruction des tissues et fibres tels que les collagènes et élastine. Ces mécanismes sont donc de manière générale responsables de la désorganisation des tissus conjonctifs.

Ainsi, les fibres de collagène natives sont spécifiquement dégradées par les métalloprotéinases (MMP), en particulier la MMP-1 dont la production est induite à partir de 0,001 DEM (Minimum Erythema Dose) c'est-à-dire l'équivalent de seulement 3 minutes d'exposition solaire. Cette production de MMP-1 évolue avec la durée et l'intensité d'exposition de manière dose - dépendante.

La perte de l'élasticité est un des problèmes majeurs de l'exposition solaire mais de manière plus générale du vieillissement cutané. Les fibres d'élastine incluant les éléments de tropoélastine et microfibrilles au niveau du derme apportent les fonctions d'élasticité. Au cours du vieillissement et en particulier sous l'action d'un stress UV, la formation des fibres élastiques et des fibres de collagène diminue et sa dégradation par les MMPs et les élastases augmentent. Les MMPs et les ROS sont par ailleurs des facteurs pro-inflammatoires qui contribuent aux dommages cutanés.

Ces mécanismes ont été largement investigués dans les domaines de la cosmétique et de la dermatologie et de nombreux agents anti-oxydants, anti-MMP, ou anti-élastase sont ainsi déjà commercialisés pour leurs effets protecteurs des tissus conjonctifs. Néanmoins la prévention et l'inhibition de la dégradation des fibres de la matrice extracellulaire, notamment des fibres élastiques et du collagène, ne sont pas des actions suffisantes pour empêcher le vieillissement intrinsèque de la peau et l'apparition de ses effets inesthétiques. En outre, aucun ingrédient cosmétique ne permet d'inhiber complètement la dégradation induite par le stress oxydant. Or, il a été observé que, sous l'effet du stress oxydant, une partie de la synthèse d'élastine dans le derme profond, est également augmentée et s'accumule de façon anarchique sous forme d'agrégat d'élastine, contribuant ainsi à la perte de l'élasticité. Par ailleurs, l'élafine est surexprimée dans le cadre d'un stress oxydatif et réticulée par la transglutaminase sur les fibres élastiques, elle contribue à la formation des agrégats d'élastine. On parle d'élastose solaire.

La Demanderesse a ainsi observé que l'amélioration des propriétés de la peau, des muqueuses et du cuir chevelu, en particulier la restauration de leur élasticité et de leur fermeté, passe notamment par l'augmentation de la qualité et/ou de la quantité des fibres de la matrice extracellulaire.

La Demanderesse avait ainsi déjà découvert que LOXL, une enzyme isoforme de la famille des lysyl oxydases (LOX) était un chaînon principal manquant dans l'élastogenèse chez l'adulte et qu'il était possible de réactiver la synthèse de cette isoforme de la lysyl oxydase afin d'obtenir un effet stimulant sur l'élastogenèse. Elle a en effet démontré sur un modèle de peau reconstruite produisant des fibres élastiques que l'activation de la synthèse de LOXL permettait de rétablir une élastogenèse fonctionnelle. La Demanderesse a par ailleurs mis également en évidence le déficit d'expression de LOXL dans les zones cicatricielles, ainsi que au niveau du derme de la peau humaine à différents âges, donc au cours du vieillissement. LOXL est l'enzyme responsable de la maturation de l'élastine par réticulation et permet donc la formation de fibres élastiques fonctionnelles. La Demanderesse a déjà identifié des actifs permettant ainsi de stimuler l'expression de LOXL (demandes de brevet FR2855968 et FR2855969, US 2004-0253220 et US 2004-0258676).

La Demanderesse a ainsi cherché un ingrédient capable à la fois d'augmenter les partenaires clé de l'élastine dans la formation des fibres élastiques, notamment LOXL et la fibuline 5 mais aussi capable d'inhiber la synthèse et/ou l'activité de l'élafine.

La recherche d'actifs alternatifs à ceux décrits dans l'art antérieur reste d'un grand intérêt dans le domaine de la cosmétique et de la dermatologie. Par ailleurs, un actif possédant la propriété d'augmenter l'expression de LOXL mais également capable d'agir sur les autres voies impliquées dans l'élastogénèse, telles que d'augmenter l'expression de la fibuline 5, de diminuer l'expression de l'élafine mais aussi d'augmenter la formation des fibres de collagènes et/ou d'améliorer leur organisation est tout particulièrement nécessaire.

La Demanderesse vient maintenant de découvrir de manière inattendue et surprenante un nouvel agent répondant à ce besoin en ce qu'il augmente la formation des fibres de la matrice extracellulaire, préférentiellement des fibres élastiques et de collagène et en ce qu'il présente plusieurs propriétés additionnelles d'un grand intérêt dans le domaine cosmétique et dans le domaine pharmaceutique pour augmenter l'élasticité et la fermeté de la peau et/ou des muqueuses et/ou du cuir chevelu, en particulier lorsque celle-ci ou celui-ci est altéré par le vieillissement chrono-induit et/ou UV induit. L'agent selon la présente invention présente en effet la propriété d'augmenter l'expression de LOXL et possède des propriétés d'inhibition de l'expression et/ou activité de l'élafine ainsi que de stimulation de la synthèse de collagène et de la fibuline 5.

Cet agent permet ainsi de fournir à lui seul, une solution complète, efficace et donc pleinement satisfaisante pour restaurer et/ou augmenter l'élasticité et/ou la fermeté des tissus altérés par le vieillissement notamment cutané et en particulier chrono- ou photo-induit. Il peut être par ailleurs utilisé en combinaison avec d'autres agents de l'art antérieur pour renforcer leurs propriétés. Cet agent permet ainsi de résoudre de manière particulièrement efficace le problème de l'art antérieur.

L'agent selon l'invention est un extrait végétal et présente ainsi l'avantage de pouvoir être facilement produit à l'échelle industrielle, d'être facilement formulable et de présenter une grande stabilité. Il est par ailleurs facilement disponible et extrait d'une ressource renouvelable, ce qui permet de le fabriquer en accord avec les principes du développement durable.

L'agent selon l'invention est un extrait de la plante *Hamamelis virginiana.*

*Hamamelis virginiana* est un arbuste originaire de l'Est de l'Amérique du Nord où il se développe dans les zones humides. Il est autrement dénommé « witch hazel » c'est-à-dire « noisetier des sorciers » car ses branches servaient autrefois à identifier l'eau et l'or, comme bâton de sourcier. *Hamamelis virginiana* est une plante couramment utilisée dans le Nord-Ouest américain pour ses propriétés astringentes et est largement disponible. Les tanins galliques et catéchiques de l'écorce pourraient être responsables de ses propriétés astringentes et hémostatiques. Elle est utilisée pour les contusions, les muscles endoloris, les varices, les hémorroïdes, les mamelons douloureux, les inflammations.
L'écorce de l'hamamélis est astringente, hémostatique, sédative et tonique. Elle est utilisée en interne (par voie orale) pour le traitement de la diarrhée, la colite, la dysenterie, les hémorroïdes, les pertes vaginales, les menstruations excessives, des hémorragies internes et des organes prolapsus. Un remède homéopathique est fabriqué à partir de l'écorce fraîche. Il est utilisé dans le traitement des saignements de nez, des hémorroïdes et des veines variqueuses.
La feuille est astringente et connue pour être utilisée comme antibactérien
Des expérimentations ont montré que l'extrait de feuille augmente la résistance des veines et diminue la perméabilité capillaire.

Un extrait d*'Hamamelis virginiana* a déjà été décrit pour des activités protectrices des tissus cutanés notamment comme agent anti-protéases notamment par inhibition des métalloprotéinases 2, 3, 1, 9 (WO200653415). Par ailleurs, des extraits sont commercialisés sous forme de distillat appelé « d*'Hamamelis virginiana* » notamment pour ses actions anti-inflammatoire, désinfectante et astringente. Néanmoins, aucune de ces propriétés connues de l'extrait d*'Hamamelis virginiana* ne laissait présager ses propriétés d'augmentation de l'élasticité et/ou de la fermeté par la formation des fibres de la matrice extracellulaire, notamment des fibres élastiques et de collagène, ni ses propriétés d'augmenter l'expression de LOXL, de la synthèse de collagène et de la fibuline 5, et/ou d'inhibition de l'expression et/ou de l'activité de l'élafine telles que découvertes dans le cadre de la présente invention.

Certaines propriétés d'un extrait végétal d*'Hamamelis virginiana* selon l'invention seront notamment démontrées plus en détails dans les exemples fournis ci-après sur son activité sur les fibres de la matrice extracellulaire notamment les fibres élastiques et les fibres de collagène.

La présente invention a donc pour objet l'utilisation cosmétique non thérapeutique d'un extrait d*'Hamamelis virginiana* pour augmenter la formation des fibres de la matrice extracellulaire dans la peau et/ou les muqueuses et/ou le cuir chevelu.

La présente invention a également pour objet l'utilisation d'un extrait d*'Hamamelis virginiana* dans une composition cosmétique, en particulier en tant qu'agent actif, pour augmenter la formation des fibres de la matrice extracellulaire dans la peau et/ou les muqueuses et/ou le cuir chevelu.

La présente invention a également pour objet l'extrait d*'Hamamelis virginiana* selon l'invention pour son utilisation par voie topique pour le soin et/ou le traitement dermatologique des pathologies impliquant les fibres élastiques et les fibres de collagène, avantageusement impliquant une perte de fermeté et/ou d'élasticité de la peau et/ou des muqueuses et/ou du cuir chevelu telles que la couperose, les télangiectasies, l'élastose solaire, la maladie cutix laxa et/ou les vergetures.

La présente invention a également pour objet une méthode ou procédé de soin et/ou de traitement cosmétique comprenant l'application par voie topique sur au moins une zone de la peau et/ou des muqueuses et/ou du cuir chevelu de l'extrait d*'Hamamelis virginiana* et/ou d'une composition cosmétique en contenant selon l'invention, pour augmenter la formation des fibres de la matrice extracellulaire dans la peau et/ou les muqueuses et/ou le cuir chevelu.

### DEFINITIONS

Selon l'invention, on entend par le terme « augmenter la formation des fibres de la matrice extracellulaire », une augmentation de la qualité et/ou la quantité des fibres de la matrice extracellulaire. Cette augmentation peut être mesurée par rapport respectivement à la qualité et/ou la quantité de fibres de la matrice extracellulaire mesurée sans application de l'extrait selon l'invention. De manière préférentielle, cette augmentation est mesurée par la visualisation et quantification de l'organisation des fibres élastiques et de collagène, comme notamment décrit par immunomarquage dans l'exemple 3. Préférentiellement, cette augmentation est au moins égale à 5% par rapport à la mesure effectuée en l'absence d'application de l'extrait selon l'invention, encore préférentiellement 10%.

Selon l'invention, on entend par le terme « fibres de la matrice extracellulaire », les protéines constitutives des fibres élastiques et/ou des fibres du collagène. Les protéines constitutives des fibres élastiques sont préférentiellement l'élastine, les fibulines, notamment la fibuline 5, et la fibrilline, notamment fibrilline 1.

Selon l'invention, on entend par « collagène », les protéines de collagène de type I, III, IV, V, VI, VII, XII, XIII, XIV, XVI, XVII, XXIV, XXIX, préférentiellement celles de la peau et/ou des muqueuses et/ou du cuir chevelu choisies parmi les collagènes I, III, IV, V et VII et préférentiellement les collagènes de type I, III et V et encore préférentiellement le collagène de type I.

Selon l'invention, on entend par « diminuer la formation d'agrégats d'élastine », l'augmentation de la synthèse de la fibuline 5 et/ou l'augmentation de l'expression de LOXL et/ou la diminution de la synthèse et/ou de l'activité de l'élafine. Un tel modèle de mesure de l'inhibition de l'expression de l'élafine est décrit dans l'exemple 4.

Selon l'invention, on entend par le terme « LOXL », ou « hLOXL » autrement dénommé LOXL-1 une isoforme de la famille des lysyl oxydases (LO), famille qui comprend 5 membres: LOX, LOXL, LOXL2, LOXL3, LOXL4 et notamment décrite dans la publication Csiszar et al. Lysyl oxidases: "A novel multifunctional amine oxidase family", Nucleic Acid Research and Molecular Biology, 2001, vol 70, p2- 28.

Selon l'invention, on entend par « augmenter l'expression de LOXL », l'augmentation de l'expression du gène codant LOXL ou de son promoteur, et notamment la stimulation de la synthèse de l'ARN messager codant LOXL, mais aussi la stimulation de la synthèse de LOXL à partir de cet ARN messager. Cette augmentation peut être mesurée sur un modèle, comprenant au moins un type cellulaire présentant une expression de LOXL, avantageusement les fibroblastes, préférentiellement cutanés, au contact de l'extrait selon l'invention et se traduit par une augmentation de l'expression génique et/ou protéique de LOXL égale ou supérieure à 10%, avantageusement égale ou supérieure à 20%, par rapport au niveau d'expression génique et/ou protéique dans un modèle témoin ou contrôle, c'est-à-dire sans mise au contact de l'extrait selon l'invention. L'augmentation de cette expression est préférentiellement génique. Un tel modèle est décrit dans l'exemple 2.

Au sens de la présente invention, on entend par « l'expression du collagène », l'expression génique du collagène, c'est-à-dire l'expression des ARNm (ARN messagers) et/ou l'expression protéique du collagène. Préférentiellement, il s'agit de l'expression protéique du collagène.

Selon l'invention, on entend par « augmenter la fermeté et/ou l'élasticité de la peau et/ou des muqueuses et/ou du cuir chevelu», une augmentation à des fins esthétiques respectivement de la fermeté et/ou élasticité de la peau et/ou des muqueuses et/ou du cuir chevelu qui ont perdu respectivement de la fermeté et/ou élasticité notamment sous l'effet de facteurs intrinsèques tels que le vieillissement tissulaire de la peau et/ou des muqueuses et/ou du cuir chevelu, c'est-à-dire le vieillissement chrono-induit, le stress cellulaire, les variations physiologiques non pathologiques telles qu'un changement de régime alimentaire, les variations hormonales, en particulier durant la puberté, la grossesse, la ménopause, l'andropause. Cette perte de fermeté et/ou d'élasticité peut également apparaitre sous l'effet de facteurs extrinsèques tels que les agents agressifs de l'environnement comme par exemple les radiations UV, la pollution, les fumées, le tabac, les toxines, les agressions climatiques et/ou mécaniques. Il peut notamment s'agir du soin et/ou traitement cosmétique de l'aspect inesthétique et/ou inconfortable des vergetures.

Au sens de la présente invention, on entend d'un point de vue dermatologique par « augmenter la fermeté et/ou l'élasticité de la peau et/ou des muqueuses et/ou du cuir chevelu », une augmentation à des fins thérapeutiques respectivement de la fermeté et/ou de l'élasticité de la peau et/ou des muqueuses et/ou du cuir chevelu qui sont sous l'effet de pathologies de la peau et/ou des muqueuses et/ou du cuir chevelu choisies parmi les télangiectasies, l'élastose solaire, la maladie cutis laxa. L'augmentation de la fermeté peut être mesurée selon les méthodes classiques, notamment par mesure in vivo par la méthode de projections de frange, à l'aide d'un cutomètre, d'un densiscore, d'un torquemètre ou encore d'un dynaskin associé à un dermatop.

L'augmentation de l'élasticité peut être mesurée selon les méthodes classiques notamment par mesure in vivo, à l'aide d'un cutomètre ou encore d'un densiscore.

Au sens de la présente invention, on entend par « utilisation et/ou composition cosmétique », une utilisation et/ou composition non pharmaceutique, c'est-à-dire qui n'est pas destinée à un usage thérapeutique et qui vise à améliorer l'esthétique et/ou le confort et qui est effectuée/appliquée sur une partie du corps saine, en particulier la muqueuse saine, la peau saine et/ou le cuir chevelu sain.

Au sens de la présente invention on entend par «peau saine», «muqueuse saine» ou «cuir chevelu sain», une zone de peau, de muqueuse ou de cuir chevelu sur laquelle est appliquée l'extrait selon l'invention et dite « non pathologique » par un dermatologue, c'est à dire ne présentant pas d'infection, de maladie ou d'affection cutanée telle que candidose, impétigo, psoriasis, eczéma, acné ou dermatite, ou de plaies ou de blessures.

Au sens de la présente invention, on entend par « cosmétique et/ou dermatologique topiquement acceptable », un ingrédient adapté à une application par voie topique, non toxique, non irritant pour la peau et/ou les muqueuses et/ou le cuir chevelu, n'induisant pas de réponse allergique, qui n'est pas instable sur le plan chimique.

Au sens de la présente invention, on entend par « application par voie topique » d'un ingrédient, l'application locale directe et/ou la vaporisation de l'ingrédient sur la surface de la peau et/ou des muqueuses et/ou du cuir chevelu.

Selon l'invention, on désigne par « muqueuse(s) », la muqueuse oculaire, la muqueuse vaginale, la muqueuse uro-génitale et/ou la muqueuse buccale, notamment buccale, labiale et/ou la muqueuse gingivale, préférentiellement, les muqueuses oculaires et/ou buccales, et encore préférentiellement, la muqueuse labiale et/ou oculaire.

### EXTRAIT

L'extrait végétal selon l'invention est un extrait des feuilles.

L'extrait peut alors être obtenu par les méthodes d'extraction de végétaux connues dans le domaine, par exemple par macération des feuilles entre 1 et 10% (p/p) dans un solvant ou un mélange de solvants, de préférence un solvant polaire protique, et avantageusement dans l'eau, un alcool, un glycol, un polyol, un mélange eau/alcool, eau/glycol ou eau/polyol (tels que l'eau en mélange avec éthanol, glycérol, butylène glycol ou autres glycols, tel que xylitol etc.) de 100/0 à 0/100 (v/v). L'extrait est préférentiellement obtenu par extraction aqueuse.

Au sens de la présente invention on entend par « extrait d*'Hamamelis virginiana* obtenu par extraction aqueuse» tout extrait obtenu par extraction avec une solution aqueuse contenant plus de 60 % en poids, avantageusement au moins 70% en poids, en particulier au moins 80% en poids, plus particulièrement au moins 90% en poids, de façon particulière au moins 95% en poids, d'eau par rapport au poids total de la solution aqueuse, encore plus avantageusement ne contenant pas de butylène glycol, en particulier ne contenant pas d'alcool, plus particulièrement ne contenant que de l'eau.

Selon un mode de réalisation avantageux, l'extrait selon l'invention est obtenu par extraction à une température comprise entre 0°C et 30°C, notamment par macération à froid, préférentiellement à 4°C, ou à température ambiante, c'est-à-dire entre 18 et 25°C, préférentiellement 20°C, éventuellement après une étape de séchage de la plante. Selon un mode préféré, l'extrait selon l'invention est obtenu par macération à température ambiante, préférentiellement 20°C. De manière avantageuse, l'extrait selon l'invention n'est pas obtenu par distillation notamment à chaud et n'est pas une huile essentielle. Encore avantageusement, l'extrait selon l'invention n'est pas obtenu à l'aide d'eau chaude ou bouillante, c'est-à-dire portée à ébullition, notamment par décoction ou par infusion.

Selon un mode de réalisation préférentielle, l'extrait selon l'invention est obtenu par macération pendant une durée entre 30 minutes et 24 heures, préférentiellement entre 1 heure et 5 heures, encore préférentiellement 2 heures.

L'extrait obtenu est ensuite de préférence centrifugé et/ou filtré afin de récupérer la fraction soluble active, préférentiellement la fraction hydrosoluble. Il est préférentiellement filtré à un seuil de coupure de 0,45µm. Des étapes supplémentaires de décoloration et/ou désodorisation peuvent être effectuées sur l'extrait à n'importe quel stade de l'extraction et selon les techniques connues par l'Homme du métier.

L'extrait selon l'invention peut également être ensuite concentré par lyophilisation ou par atomisation.

De façon particulièrement avantageuse, la quantité de plante lors de l'extraction, soit les feuilles, est comprise entre 1 et 10% en poids, préférentiellement de 5 % en poids par rapport au poids total du mélange plante/solvant d'extraction, préférentiellement la solution aqueuse. En particulier la plante est broyée avant l'extraction.

De façon avantageuse la durée de l'extraction est de 2 heures et effectuée à température ambiante, préférentiellement 20 °C.

L'extrait obtenu selon l'invention est préférentiellement soluble et dissous dans un solvant notamment polaire, tel que l'eau, un alcool, un polyol, un glycol, ou un de leurs mélanges, préférentiellement un mélange hydroglycolique, encore préférentiellement contenant un glycol choisi parmi le caprylyl glycol, l'hexylène glycol, le pentylène glycol et leurs mélanges.

Avantageusement l'extrait selon l'invention est dissous dans une solution aqueuse contenant du pentylène glycol et/ou du caprylyl glycol, en particulier contenant entre 0,01 et 10 % en poids de pentylène glycol et/ou de caprylyl glycol par rapport au poids total la solution aqueuse, plus particulièrement entre 0,1 et 5 % en poids du pentylène glycol et/ou du caprylyl glycol par rapport au poids total la solution aqueuse.

### UTILISATION COSMETIQUE

La présente invention a ainsi pour objet l'utilisation cosmétique de l'extrait d*'Hamamelis virginiana* pour améliorer la formation des fibres de la matrice extracellulaire dans la peau, les muqueuses et/ou le cuir chevelu et préférentiellement la formation des fibres élastiques et/ou les fibres de collagène.

Par les propriétés sur l'augmentation de l'expression de LOXL, de l'expression de la fibuline 5, et d'inhibition de la synthèse et/ou de l'activité de l'élafine, l'extrait selon l'invention augmente globalement l'élastogénèse et ainsi peut être utilisé pour augmenter l'élasticité de la peau et/ou des muqueuses et/ou du cuir chevelu.

De manière particulièrement avantageuse, l'extrait d*'Hamamelis virginiana* utilisé dans la présente invention, en particulier via ses propriétés sur l'inhibition de la synthèse et/ou activité de l'élafine permet également de diminuer la formation d'agrégats d'élastine. En outre, l'extrait d*'Hamamelis virginiana* selon l'invention par ses propriétés d'augmentation de l'expression et/ou l'activité du collagène, peut être utilisé pour augmenter la fermeté de la peau et/ou des muqueuses et/ou du cuir chevelu.

L'extrait d*'Hamamelis virginiana* selon l'invention peut être utilisé, notamment sous la forme d'un ingrédient actif cosmétique ou dermatologique, en tant que tel, ou contenu dans une composition cosmétique ou dermatologique contenant un excipient cosmétique et/ou dermatologique topiquement acceptable.

Ladite composition cosmétique ou dermatologique est utilisée avantageusement en application par voie topique sur la peau et/ou les muqueuses et/ou le cuir chevelu.

De manière préférentielle, l'extrait d*'Hamamelis virginiana* selon l'invention est contenu dans la composition cosmétique ou dermatologique à une concentration comprise entre 1.10⁻⁴ et 10% en poids, et avantageusement entre 1.10⁻⁴ et 5% et plus particulièrement entre 1.10⁻³ et 3% en poids par rapport au poids total de la composition.

Les compositions cosmétiques ou dermatologiques selon l'invention contiennent donc un excipient cosmétique ou dermatologique topiquement acceptable en plus de l'extrait selon l'invention. Cet excipient est par exemple au moins un composé choisi dans le groupe consistant en les conservateurs, les émollients, les émulsifiants, les tensioactifs, les hydratants, les épaississants, les conditionneurs, les agents matifiants, les stabilisants, les antioxydants, les agents de texture, les agents de brillance, les agents filmogènes, les solubilisants, les pigments, les colorants, les parfums et les filtres solaires. Ces excipients sont de préférence choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxidases, les stabilisants à base de sucrose, les vitamines E et ses dérivés, les cires naturelles et synthétiques, les huiles végétales, les triglycérides, les insaponifiables, les phytostérols, les esters végétaux, les silicones et ses dérivés, les hydrolysats de protéines, l'huile de Jojoba et ses dérivés, les esters lipo/hydrosolubles, les betaines, les aminoxides, les extraits de plantes, les esters de Saccharose, les dioxydes de Titane, les glycines, et les parabens, et encore de préférence parmi le groupe consistant en le stéareth-2, le stéareth-21, le glycol-15 stéaryle éther, le cétéaryl alcool, le phénoxyéthanol, le méthylparaben, l'éthylparaben, le propylparaben, le butylparaben, le butylène glycol, le caprylyl glycol, les tocophérols naturels, la glycérine, le dihydroxycetyl sodium phosphate, l'isopropyl hydroxycétyl éther, le glycol stéarate, le triisononanoin, l'octyl cocoate, le polyacrylamide, l'isoparaffine, le laureth-7, un carbomer, le propylène glycol, l'hexylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, Beeswax, les glycérides d'huile de coeur de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée.

La composition cosmétique selon l'invention peut être choisie parmi une solution, aqueuse ou huileuse, une crème ou un gel aqueux ou un gel huileux, notamment un gel douche, un shampoing ; un lait ; une émulsion, une microémulsion ou une nanoémulsion, notamment huile-dans-eau ou eau-dans-huile ou multiple ou siliconée ; un masque ; un sérum ; une lotion ; un savon liquide ; un pain dermatologique ; une pommade ; une mousse ; un patch ; un produit anhydre, de préférence liquide, pâteux ou solide, par exemple sous forme de poudres de maquillage, de bâtonnet ou de stick, notamment sous forme de rouge à lèvre. De façon avantageuse il s'agit d'une crème ou d'un sérum, en particulier un contour des yeux ou des lèvres.

La composition cosmétique selon l'invention peut notamment être choisie parmi les soins solaires, les soins anti-âges, notamment pour les peaux dites matures, et/ou des peaux présentant les premiers signes de vieillissement,

Au sens de la présente invention, on entend par « peaux matures », les peaux des femmes ou hommes ayant au minimum 50 ans, avantageusement les peaux des femmes ménopausées.

Au sens de la présente invention, on entend par « peaux présentant les premiers signes de vieillissement », les peaux des femmes ou hommes ayant entre 30 et 40 ans, avantageusement les peaux présentant les premières rides d'expression.

Les compositions selon l'invention sont plus particulièrement appliquées au niveau du visage, en particulier en contour des yeux ou des lèvres, préférentiellement quotidiennement, préférentiellement une à deux fois par jour, préférentiellement le matin et/ou le soir.

Avantageusement, les compositions cosmétiques selon l'invention contiennent d'autres ingrédients d'intérêt notamment cosmétique, préférentiellement des agents ayant des propriétés similaires. Préférentiellement il s'agit des ingrédients classiques des compositions anti-âges et/ou améliorant l'élasticité et/ou la fermeté de la peau et/ou des muqueuses et/ou du cuir chevelu et/ou améliorant le teint de la peau et/ou l'homéostasie cutanée notamment ceux choisis parmi les agents de comblement, les agents hydratants, les agents stimulants les molécules de la matrice extracellulaire.

Les compositions cosmétiques selon l'invention peuvent également contenir des ingrédients actifs cosmétiques conduisant à un effet complémentaire ou éventuellement synergique tels que des actifs hydratants, des actifs anti-âge, des actifs anti-radicalaires, les agents protecteurs du facteur de croissance des fibroblastes (FGF), les agents stimulant l'activité et/ou la prolifération des fibroblastes et/ou des eaux thermales. Il peut ainsi s'agir par exemple d'agents de coloration de la peau ou pro-pigmentants, d'inhibiteurs de NO-synthase, d'agents anti-séborrhéiques pour le soin des peaux grasses, d'agents stimulant la synthèse de macromolécules dermiques ou épidermiques, notamment de la matrice extracellulaire et/ou empêchant leur dégradation, pour un effet synergique ou complémentaire, d'agents stimulant la prolifération des fibroblastes ou des kératinocytes et/ou la différenciation des kératinocytes, pour un effet synergique ou complémentaire, d'agents antimicrobiens, d'agent tenseurs, d'agents antipollutions ou anti-radicalaires, d'agents apaisants, calmants ou relaxants, d'agents agissant sur la microcirculation pour améliorer l'éclat du teint, en particulier du visage, pour un effet synergique ou complémentaire, d'agents photoprotecteurs, d'agents cicatrisants, d'agents amincissants, d'agents anti âge pour un effet synergique ou complémentaire ou éventuellement d'agents hydratants et/ou renforçant la barrière épidermique.

Les actifs hydratants, émollients ou humectants peuvent renforcer la fonction barrière et diminuer les pertes insensibles en eau et/ou augmenter la teneur en eau de la peau et/ou des muqueuses et/ou du cuir chevelu ou stimuler l'activité sécrétoire des glandes sébacées et/ou stimuler la synthèse d'aquaporine pour améliorer la circulation de l'eau dans les cellules. On peut citer à titre d'exemple non limitatif les actifs suivants : la sérine, l'urée et ses dérivés, les produits commercialisés sous le nom de Marine Filling sphères™, Advances moisturizing complex™, Hyaluronic Filling Spheres™, vegetal filling spheres™ Osmogelline™, Micropatch™, les alkylcelluloses, les lécithines, les composés à base de sphingoïde, les céramides, les phospholipides, le cholestérol et ses dérivés, les glycosphingolipides, les phytostérols (stigmastérol et béta-sitostérol, campestérol), les acides gras essentiels, le 1-2 diacylglycérol, la 4-chromanone, les triterpènes pentacycliques tels que l'acide ursolique, la vaseline, la lanoline, les sucres en particulier le tréhalose et ses dérivés, le rhamnose, fructose, maltose, lactose, erythritol, le mannitol, le D-xylose et le glucose, l'adénosine et ses dérivés, le sorbitol, les alcools polyhydriques, avantageusement en C2-C6, et de façon encore avantageuse en C3-C6, tels que la glycérine, le propylène glycol, le dipropylène glycol, la diglycérine, la polyglycérine et leur mélange, le glycérol et ses dérivés, le polyacrylate de glycérol, le lactate de sodium, le pentanediol, la serine, les acides lactiques, les AHA, les BHA, le pidolate de sodium, le xylitol, le lactate de sodium, l'ectoine et ses dérivés, le chitosane et ses dérivés, le collagène, le plancton, les dérivés stéroïdiens (dont la DHEA, ses dérivés 7-oxydés et/ou 17-alkylés et les sapogénines), le dihydrojasmonate de méthyle, , un extrait de Malva sylvestres ou un extrait de Centella asiatica, des homopolymères d'acide acrylique, le beta-glucan et en particulier le sodium carboxymethyl beta-glucane, un dérivé C-glycoside tel que ceux décrits dans la demande WO 02/051828, une huile de rosier muscat, un extrait de micro-algue Prophyridium cruentum enrichi en zinc commercialisé par Vincience sous la dénomination Algualane Zinc™, l'arginine, l'acetyl hexapeptide commercialisé par Lipotech sous le nom Diffuporine™, l'hydrolysat de Viola tricolor commercialisé par Silab sous le nom Aquaphyline™.

L'actif peut également être choisi parmi les agents anti-âge, c'est-à-dire ayant notamment un effet restructurant de la barrière cutanée, les agents prévenant et/ou diminuant la glycation des protéines de la peau en particulier des protéines du derme, telles que le collagène, les actifs stimulant le métabolisme énergétique des cellules et leurs mélanges, un agent à action globale anti-âge, en particulier la niacinamide ou vitamine B3 et dérivés. L'agent ayant un effet restructurant de la barrière cutanée peut être choisi parmi un des extraits de levure comme le Relipidium™ de BASF Beauty Care Solutions France SAS, des sphingosines comme la salicyloyl sphingosine, un mélange de xylitol, de xylityl polyglycoside et de xylitan, des extraits de solanacée comme le Lipidessence™ de BASF Beauty Care Solutions France SAS et leurs mélanges. On peut encore citer notamment les céramides, les composés à base de sphingoïdes, les glycosphingolipides, les phospholipides, le cholestérol et ses dérivés, les phytostérols, les acides gras essentiels, le diacylglycérol, la 4-chromanone et dérivés de chromone et leurs mélanges, vitamine B5 ou pantothenate et dérivés.

L'actif stimulant le métabolisme énergétique des cellules peut par exemple être choisi parmi la biotine, un mélange de sels de sodium, de manganèse, de zinc et de magnésium d'acide pyrrolidone carboxylique, un mélange de gluconate de zinc, de cuivre et de magnésium et leurs mélanges.

Parmi les actifs stimulant la synthèse des macromolécules du derme ou empêchant leur dégradation, on peut citer ceux qui agissent comme :
- un agent stimulant la synthèse de fibronectine, en particulier un extrait de maïs, un tel extrait étant notamment commercialisé par la Demanderesse sous le nom Deliner™ et le palmitoyl pentapeptide commercialisé par la société SEDERMA sous la dénomination commerciale Matrixil™,
- un agent de protection du facteur de croissance des fibroblastes (FGF2) de la matrice extracellulaire contre sa dégradation et/ou sa dénaturation, notamment un extrait d'Hibiscus abelmoscus tel que décrit dans la demande de brevet au nom de la Demanderesse publiée sous le numéro FR2907014 et/ou un agent de stimulation de croissance des fibroblastes par exemple un extrait de soja fermenté contenant des peptides, connu sous le nom de Phytokine™ commercialisé par la Demanderesse et également décrit dans la demande de brevet EP1119344 (Laboratoires Expanscience), et préférentiellement une combinaison de ces deux extraits;
- un agent stimulant la synthèse de laminine, en particulier un extrait de malt modifié par biotechnologie, un tel extrait étant notamment commercialisé par la Demanderesse sous le nom Basaline™ ;
- un agent stimulant l'expression et/ou l'activité de la Hyaluronane synthase 2 (HAS2) tels que les extraits végétaux décrits dans la demande de brevet FR2 893 252 A1 et en particulier un extrait aqueux de Galanga (Alpinia galanga) ;
- un agent stimulant la synthèse de lysyl oxydase like (LOXL) tel que un extrait de Geophila cordifolia et ceux décrits dans la demande de brevet FR2855968, et en particulier un extrait d'aneth ;
- un agent stimulant la synthèse d'ATP intracellulaire, notamment un extrait d'algue Laminaria digitata ;
- un actif stimulant la synthèse des glycosaminoglycanes, tels que le produit de fermentation du lait ;
- un actif stimulateur de collagène tel que le rétinol et/ou la vitamine C ;
- un actif inhibiteur des métalloprotéinases (MMP) telles que plus particulièrement les MMP 1, 2, 3, 9 et 12 tel que les rétinoïdes et dérivés, les oligopeptides et les lipopeptides, les lipoaminoacides, l'extrait de malt commercialisé par BASF Beauty Care Solutions France sous la dénomination commerciale Collalift™, l'extrait hydrolysé de pomme de terre commercialisé sous le nom Extracellium™ par BASF Beauty Care solutions France SAS; le lycopène ; les isoflavones, la quercetine, le kaempferol, l'apigenine.

Les agents stimulant la prolifération des kératinocytes, utilisables dans la composition selon l'invention, comprennent notamment les rétinoïdes tels que le rétinol et ses esters, dont le palmitate de rétinyle et le phloroglucinol. Les agents stimulant la différenciation des kératinocytes comprennent par exemple les minéraux tels que le calcium et les lignanes tels que le sécoisolaricirésinol ainsi que l'extrait d'Achillea millefollium commercialisé sous le nom de Neurobiox™ par BASF Beauty Care Solutions France.

Parmi les agents tenseurs utilisables dans la composition selon la présente invention, on peut citer notamment les polymères synthétiques, tels que les latex de polyuréthanne ou les latex acryliques ; les polymères d'origine naturelle, notamment les polyholosides sous forme d'amidon ou sous forme de carraghénanes, alginates, agars, gellanes, polymères cellulosiques et pectines; les protéines et hydrolysats de protéines végétales de soja ; les silicates mixtes ; les microparticules de cire ; les particules colloïdales de charge inorganique choisies par exemple parmi la silice, les composites silice-alumine ; ainsi que leurs mélanges.

La composition peut comprendre des agents dits antipollution, en particulier piégeur d'ozone que sont par exemple la vitamine C et ses dérivés dont le glucoside d'ascorbyle; les phénols et polyphénols, en particulier les tannins, l'acide ellagique et l'acide tannique; l'épigallocatéchine et les extraits naturels en contenant, en particulier les extraits de thé vert; les anthocyanes; les acides phénols, les stilbènes; des actifs piégeurs de composés aromatiques mono- ou polycycliques les tannins tels que l'acide ellagique et les dérivés indoles et/ou des actifs piégeurs de métaux lourds tels que l'EDTA, des actifs anti-radicalaires tels que la vitamine E et ses dérivés tels que l'acétate de tocophéryle ; les bioflavonoïdes; la coenzyme Q10 ou ubiquinone.

Comme agents apaisants utilisables dans la composition selon l'invention, on peut citer : les triterpènes pentacycliques, l'acide ursolique et ses sels, l'acide oléanolique et ses sels, l'acide bétulinique et ses sels, les sels de l'acide salicylique et en particulier le salicylate de zinc, le bisabolol, l'allantoïne, les huiles insaturées en oméga 3, la cortisone, l'hydrocortisone, l'indométhacine et la beta méthasone, les actifs anti-inflammatoires, et notamment ceux décrits dans la demande FR2847267, en particulier l'extrait de racine de Pueraria lobata commercialisé sous le nom Inhipase™ par BASF Beauty Care Solutions France SAS, les extraits de Theobroma cacao.

Les ingrédients actifs photoprotecteurs ou filtres UVA et/ou UVB utilisables selon la présente invention sont notamment les agents photoprotecteurs actifs dans l'UV-A et/ou l'UV-B, tels que les dérivés de l'acide para-amino-benzoïque notamment UVINUL P25™ commercialisé par BASF, les dérivés salicyliques en particulier l'homosalate seul ou en association avec des oxydes de titane, les dérivés du dibenzoylméthane, les dérivés cinnamiques, les dérivés de diphénylacrylate, dont Octocrylene vendu notamment sous le nom commercial UVINUL N539™ par BASF, les dérivés de la benzophénone, notamment Benzophenone-1 vendue notamment sous le nom commercial UVINUL 400™ par BASF, les dérivés du benzylidène camphre, les dérivés de benzimidazole, les dérivés de triazine, dont Ethylhexyl triazone vendu notamment sous le nom commercial UVINUL T150™ par BASF, les dérivés de benzotriazole, les dérivés anthranilique, les dérivés d'imidazolines les dérivés de benzalmalonate, les dérivés de 4,4-diarylbutadiène, et leurs mélanges.

Les actifs procurant un effet de bien-être tels que ceux mimant les effets des béta-endorphines pour améliorer la fonction barrière de la peau, tels que ceux cités dans la demande de brevet US 2006069032 ; les actifs stimulants la synthèse des béta-endorphines tels que un extrait de la plante Tephrosia purpurea.

Les actifs amincissants peuvent être notamment choisis parmi : les agents inhibiteurs de la lipoprotéine lipase tels que ceux décrits dans le brevet US2003086949 (Coletica) et en particulier un extrait de liane du Pérou (Uncaria tomentosa); les actifs drainants, notamment l'hesperitine laurate (Flavagrum™), or quercitine caprylate (Flavenger™); les agents inhibiteurs de l'enzyme phosphodiestarase, les agents activateurs de l'adenylate cyclase, l'AMPc et/ou les actifs capable de piéger la spermine et/ou la spermidine. On peut citer à titre d'exemple de ces actifs un extrait de racine de Coleus Forskohlii, un extrait de cecropia obtusa, d'Uva lactuca, la caféine, la forskoline, la théophylline, la théobromine et/ou leurs dérivés, un produit de kappa carraghénanes hydrolysé dénommé Slimexcess™ commercialisé par BASF Beauty Care Solutions France SAS et/ou leurs mélanges.

De nombreux ingrédients cosmétiquement actifs sont connus par l'homme du métier pour améliorer la santé et/ou l'apparence physique de la peau et/ou les muqueuses et/ou du cuir chevelu. L'homme du métier sait formuler les compositions cosmétiques pour obtenir les meilleurs effets.

D'autre part les composés décrits dans la présente invention peuvent avoir un effet de synergie lorsqu'ils sont combinés les uns aux autres. Ces combinaisons sont également couvertes par la présente invention.

Le CTFA Cosmetic Ingredient Handbook, Second Edition (1992) décrit différents ingrédients cosmétiques et pharmaceutiques utilisés couramment dans l'industrie cosmétique, qui sont en particulier adaptés à une utilisation topique. Des exemples de ces classes d'ingrédients comprennent, sans en être limité les composés suivants: abrasif, absorbants, composé à but esthétique tel que les parfums ; les pigments ; les colorants ; les huiles essentielles ;les astringents tels que l'huile de clou de girofle, menthol, camphre, l'huile d'eucalyptus, eugénol, menthyl lactate, distillat d'hamélis ; les agents anti-acné ; les agents anti-floculants ; les agents antimousse ; les agents antimicrobiens tels que iodopropyl butylcarbamate ; les antioxydants tels que l'acide ascorbique; les liants ; les additives biologiques ; les agents tampon ; les agents gonflants ; les agents chélatants ; les additifs ; les agents biocides ; les dénaturants ; les épaississants ; et les vitamines ; les matériaux formant des films ; les polymères ; les agents opacifiants ; les ajusteurs de pH ; les agents réducteurs ; les agents de conditionnement tels que les humectants, et les dérivés ou équivalents de ceux-ci.

De manière préférentielle, les compositions selon l'invention contiennent moins de deux composés choisis parmi la cyclodextrine, le pentetate de pentasodium, l'acide phytique, le citrate de potassium, le citrate de sodium, le gluconate de potassium et le gluconate de sodium, avantageusement elles ne contiennent pas de composé choisi parmi la cyclodextrine, le pentetate de pentasodium, l'acide phytique, le citrate de potassium, le citrate de sodium, le gluconate de potassium et le gluconate de sodium.

La présente invention a également pour objet un procédé de soin et/ou traitement cosmétique caractérisé en ce qu'il comprend l'application par voie topique sur au moins une zone de peau et/ou muqueuses et/ou du cuir chevelu d'un extrait d*'Hamamelis virginiana* pour augmenter la formation des fibres de la matrice extracellulaire dans la peau et/ou les muqueuses et/ou le cuir chevelu, préférentiellement les fibres élastiques et/ou les fibres de collagène.

Le procédé de soin et/ou traitement cosmétique selon l'invention permet d'augmenter l'expression de LOXL, l'expression et/ou l'activité du collagène, l'expression et/ou l'activité de la fibuline 5, et/ou d'inhiber de l'expression et/ou activité de l'élafine et est donc particulièrement utile pour diminuer la formation d'agrégats d'élastine et de manière plus générale pour augmenter la fermeté et/ou l'élasticité de la peau et/ou de muqueuses et/ou du cuir chevelu.

Selon un mode particulièrement avantageux, le procédé de soin et/ou traitement cosmétique est appliqué sur une zone de peau et/ou muqueuse choisie parmi au moins le cuir chevelu, le cou, le décolleté, le ventre, les bras, les cuisses, les hanches, les fesses, la taille et/ou le visage, et préférentiellement le visage, en particulier le contour des yeux, les lèvres. L'invention a également pour objet l'extrait d*'Hamamelis virginiana* selon l'invention pour son utilisation par voie topique pour le soin et/ou le traitement dermatologique des pathologies impliquant une perte de fermeté et/ou d'élasticité de la peau et/ou des muqueuses et/ou du cuir chevelu choisies parmi les télangiectasies, l'élastose solaire, la maladie cutix laxa. De manière avantageuse, l'extrait d*'Hamamelis virginiana* selon l'invention est présent dans une composition dermatologique comprenant un excipient dermatologique topiquement acceptable.

De manière préférentielle, l'extrait selon l'invention est contenu à une concentration comprise entre 1.10⁻⁴ et 10% en poids, et avantageusement entre 1.10⁻⁴ et 5% et plus particulièrement entre 1.10⁻³ et 3% en poids par rapport au poids total de la composition dermatologique.

D'autres buts, caractéristiques et avantages de l'invention apparaîtront clairement à l'homme de l'art suite à la lecture de la description explicative qui fait référence aux figures et à des exemples qui sont donnés seulement à titre d'illustration et qui ne sauraient en aucune façon limiter la portée de l'invention.
La FIGURE 1 représente la visualisation en immunomarquage de la Fibuline 5 avec ou sans extrait selon l'invention sur culture de fibroblastes humains (A : témoin non traité ; B : cellules traitées en présence d'un extrait d*'Hamamelis virginiana* selon l'invention à 0,1% (v/v) par rapport au milieu de culture).
La FIGURE 2 représente la visualisation en immunomarquage de l'élastine avec ou sans extrait selon l'invention sur culture de fibroblastes humains (A : témoin non traité ; B : cellules traitées en présence d'un extrait d*'Hamamelis virginiana* selon l'invention à 0,05% (v/v) par rapport au milieu de culture).
La FIGURE 3 représente la visualisation en immunomarquage du collagène type I sur culture de fibroblastes humains. (A : témoin non traité ; B : cellules traitées en présence d'un extrait d*'Hamamelis virginiana* selon l'invention à 0,1% (v/v) par rapport au milieu de culture).
La FIGURE 4 représente la visualisation en immunomarquage de l'élafine sur modèle de peau reconstruite. (4a : Témoin Négatif, 4b : Témoin Irradié, 4c : Peau Traitée en présence d'un extrait d*'Hamamelis virginiana* selon l'invention à 0,1% (v/v) par rapport au milieu de culture).

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

D'autre part, dans les exemples, et sauf indication contraire, la température est exprimée en degré Celsius, la pression est la pression atmosphérique.

### Exemple 1 : préparation d'extrait d'Hamamelis virginiana selon l'invention par extraction aqueuse.

a) Les parties aériennes, ici les feuilles, sont broyées puis mises à macérer dans l'eau pendant 2 heures à température ambiante c'est-à-dire entre 18 et 25°C, ici à environ 20°C, la teneur en feuilles d*'Hamamelis virginiana* broyées étant de 5% en poids par rapport au poids total plante/eau.
   Les insolubles sont séparés par centrifugation à 8000 tours par minute (rpm) et par filtration à 0,45µm et on récupère le liquide qui contient l'extrait aqueux selon l'invention. Le pH a été mesuré et ajusté à une valeur allant de 5 à 7,5. Cet extrait a été testé à différents dosages dans le milieu de culture final dans les exemples suivants.
   Cet extrait peut également être formulé sous la forme d'un ingrédient cosmétique tel que présenté en exemple 5.
b) Les feuilles d*'Hamamelis virginiana* sont broyées puis mises à macérer dans l'eau à 5% (p/p), à une température préférentiellement comprise entre 0 et 20°C, préférentiellement à 4°C.
   La durée de macération est avantageusement comprise entre 30min et 24heures, sous agitation, ici 16 heures.
   La solution est centrifugée, préférentiellement pendant 10min à 8000 tours par minute (rpm) et le surnageant est récupéré. Le surnageant est ultrafiltré sur filtres à différents seuil de coupure et notamment à 0,45 µm.
   L'extrait ainsi obtenu peut être utilisé directement sous forme liquide.
c) Les feuilles d*'Hamamelis virginiana* sont broyées puis mises à macérer à 5% (p/p) dans un mélange eau/butylène glycol à 75%/25% (v/v) à une température préférentiellement comprise entre 0 et 20°C, ici à 4°C. La durée de macération est avantageusement comprise entre 30min et 24heures, sous agitation, ici 16 heures.
   La solution est centrifugée, préférentiellement pendant 10min à 8000 tours par minute (rpm) et le surnageant est récupéré. Le surnageant est ultrafiltré sur filtres à différents seuil de coupure et notamment à 0,45µM.
   L'extrait ainsi obtenu est ensuite séché notamment sur support de type maltodextrine puis resolubilisé dans de l'eau à 1% (p/p).

### Exemple 2 : Mise en évidence de la propriété d'augmentation de l'expression du gène LOXL de l'extrait d'Hamamelis virginiana selon l'invention.

### Principe :

L'expression du gène de LOXL a été mesurée par la méthode de RT-PCR quantitative dans des conditions de culture standard. Son niveau d'expression a été rapporté au nombre de cellules grâce au gène de ménage ACTINE.

### Protocole expérimental :

### 1) Protocole de culture :

Des fibroblastes humains normaux (NHF) issus de biopsie abdominale d'un donneur de 62 ans ont été cultivés en monocouche à 37°C avec 5% de CO₂ dans un milieu spécifique à la culture des fibroblastes.

Les cellules en P8 ont été ensemencées à 25 000 cellules par cm² en plaques 24 puits, avec pour chaque condition N=4, en milieu FGM (fibroblast growth médium, Promocell) spécifique à la croissance des fibroblastes.

### 2) Application de l'actif testé

L'extrait d*'Hamamelis virginiana* obtenu à l'exemple 1a) a été appliqué pendant 24h à la concentration finale dans le milieu de culture testée (de 0,01% (v/v)) sur les cellules à confluence dans le milieu de culture FGM complet. Le traitement des cellules est arrêté par un rinçage en PBS et les plaques sont congelées à sec à -80°C, en attendant l'extraction des ARNs totaux.

Un contrôle non traité a été effectué avec le milieu FGM seul.

### 3) Extraction des ARN totaux

Les ARN totaux des fibroblastes cultivés en monocouche ont été extraits (kit SV96 total RNA isolation system (Promega)), quantifiés par spectrophotométrie à 260/280 nm et congelés à -80°C pour la Q RT PCR ci-dessous.

### 4) Réaction quantitative en chaine par polymérase après transcription inverse (Q RT PCR)

La réaction d'amplification en chaîne est réalisée avec le kit Quantitect SYBR Green Kit® (Qiagen, USA). Les amorces utilisées sont détaillées dans le tableau 1. Un mix PCR de 50 µL contenant 10 µL d'ARNs à 5 ng/mL, 25 µL de mix SybrGreen, 0,5 µL de mix enzymatique et 0,625 µL de chaque amorce à 400 µM qsp eau a été préparé dans les plaques PCR (après décongélation de celles-ci). Puis la plaque PCR a été centrifugée pendant 1 min à 1500 rpm.

Les ARN messagers spécifiques du gène d'intérêt et du gène de l'actine ont ensuite été amplifiés et quantifiés par RT-PCR quantitative en présence des amorces spécifiques de chaque gène (tableau 1).

**Tableau 1 : séquence d'amorces utilisées pour la RT-PCR**

| Gène | Longueur de l'ADN amplifié (pb) | Longueur de l'ADN génomique (pb) | Température d'amplicon attendue (°C) | amorce | Séquence |
|---|---|---|---|---|---|
| Actine | 540 | 1114 | 86-87 | Sens | 5'-gtg ggg cgc ccc agg cac ca-3' (SEQ ID n°1) |
| | | | | Anti-sens | 5'-ctc ctt att gtc acg cac gat ttc-3' (SEQ ID n°2) |
| LOXL | 240 | 2359 | 82-83 | Sens | 5'-gac ttc ggc aac ctc aag c-3' (SEQ ID n°3) |
| | | | | Anti-sens | 5'-tgt tgc aga aac gta gcg ac-3' (SEQ ID n°4) |

### 5) Analyse des résultats :

Les résultats bruts obtenus après Q-RT-PCR ont été rapportés à l'expression de l'actine afin de normaliser les résultats. Puis les résultats normalisés ont été rapportés à la valeur moyenne du contrôle non traité afin de mesurer les régulations d'expression.

### Résultats :

Les résultats sont exprimés en induction du gène LOXL par rapport au contrôle non traité par l'extrait 1 a).

**Tableau 2 :**

| Actif testé | Induction des ARNm LOXL | Déviation standard |
|---|---|---|
| Contrôle non traité | 1,00 | 0,47 |
| Extrait à 0,01% (v/v) par rapport au milieu de culture | 2,09 | 0,26 |

### Conclusions

Sous l'effet de l'extrait selon l'invention, une augmentation de l'expression du gène de LOXL a été observée puisque la synthèse d'ARNm de LOXL est augmentée par rapport au témoin. Ceci témoigne de l'efficacité de l'extrait selon l'invention sur l'augmentation de l'expression de LOXL et sa capacité à être utilisé pour augmenter la formation des fibres élastiques et la fermeté.

### Exemple 3 : Visualisation de l'augmentation de la formation des fibres élastiques et de collagène :

### Protocole :

Les fibroblastes issus d'une biopsie abdominale d'un donneur de 62 ans ont été cultivés sur lames de verre (labteks) en passage 8 en milieu défini FGM (Promocell) jusqu'à confluence. Le jour de la confluence, les cellules ont été traitées par l'actif dans du milieu FGM seul ou additionné de vitamine C (5µg/mL). Le milieu a été renouvelé tous les 2 jours durant 3 jours pour l'analyse du collagène et 6 jours pour l'analyse des fibres élastiques (élastine et fibuline 5). L'extrait d*'Hamamelis virginiana* obtenu selon l'exemple 1a) et dilué à 0,1% par rapport au milieu de culture (v/v) dans le milieu de culture a été testé et réappliqué à chaque changement de milieu. Le témoin non traité correspond au milieu de culture seul additionné de 5 µg/mL de vitamine C dans le cas du collagène ou de 0,1ng/mL de TGFβ 1 pour l'élastine et la fibuline 5. En fin de culture, le milieu a été retiré, les cellules rincées en tampon phosphate salin (PBS) puis fixées en méthanol à -20°C afin de procéder à l'immunomarquage.

Les marquages ont été ici réalisés par technique d'immunofluorescence. Après rinçage du méthanol au PBS, les sites antigéniques aspécifiques sont alors bloqués 1 heure avec une solution de PBS-BSA 1% (albumine sérique bovine). L'anticorps primaire dilué dans du PBS/BSA 1% est déposé sur les lames et mis à incuber pendant 1 heure. Sur le témoin négatif est déposé un isotype contrôle IgG (à la place de l'anticorps primaire). L'excès d'anticorps non fixé est éliminé par deux rinçages au PBS.

Les cellules sont incubées à l'abri de la lumière avec une solution d'anticorps secondaires capables de reconnaitre l'anticorps primaire. Cet anticorps secondaire étant couplé à un fluorochrome (Alexa Fluor 488), il est ainsi possible de visualiser la protéine en question sous un microscope à fluorescence. Les lames sont ensuite lavées par 3 bains de PBS et montées avec un milieu de montage contenant un marqueur nucléaire, du DAPI (4',6'-diamidino-2-phénylindole) (Invitrogen). Enfin, les lames peuvent être observées au microscope confocal.

**Tableau 3 : anticorps utilisés pour l'immunomarquage**

| Anticorps | Hôte | Dilution |
|---|---|---|
| Anti-Collagène de type I | Lapin | 1/200 |
| Anti-Fibuline 5 | Lapin | 1/500 |
| Elastine | Lapin | 1/100 |
| Anti-lgG de lapin couplé AlexaFluor 488 | Chèvre | 1/1000 |
| Anti-lgG1 de souris couplé AlexaFluor 488 | Chèvre | 1/1000 |

### Résultats :

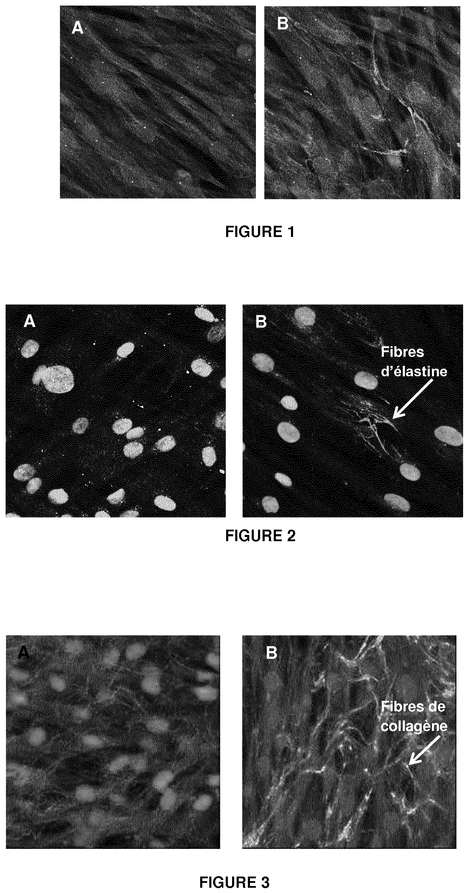

Les résultats sont présentés dans les figures 1, 2 et 3.

### 3a) Quantification de la longueur des fibres positives pour la fibuline 5 (µm) :

| Condition | Moyenne | Ecart type |
|---|---|---|
| Témoin non traité | 431 | 105 |
| Extrait selon l'invention à 0,1% (v/v) | 1106 | 64 |

L'observation en microscopie confocale est présentée dans la figure 1.

### Conclusion :

La fibuline 5 est localisée sur les fibres élastiques lorsque la culture a été effectuée en présence de l'extrait d*'Hamamelis virginiana* selon l'invention (Figure 1B) alors qu'elle est diffuse lorsque la culture a été effectuée sans l'extrait (témoin non traité) (Figure 1A). Ceci démontre que l'extrait selon l'invention augmente la formation des fibres élastiques notamment par une meilleure qualité de celles-ci en les organisant en fibres élastiques matures.

### 3b) Quantification de la longueur des fibres positives pour l'élastine (µm) :

| Condition | Moyenne | Ecart type |
|---|---|---|
| Témoin non traité | 32,5 | 10,9 |
| Extrait selon l'invention à 0,05% (v/v) | 772,2 | 201,9 |

L'observation en microscopie confocale est présentée dans la figure 2.

### Conclusion :

L'élastine apparait sous forme fibrillaire lorsque la culture a été effectuée en présence de l'extrait d*'Hamamelis virginiana* selon l'invention (Figure 2B) alors qu'elle apparait sous une forme globulaire, non organisée en l'absence de l'extrait (témoin non traité) (Figure 2A). Ceci démontre que l'extrait selon l'invention augmente la formation des fibres élastiques notamment par une meilleure qualité de celles-ci en les organisant en fibres élasiques matures.

### 3c) Quantification de l'augmentation de la formation des fibres de collagène de type I (en pourcentage de la valeur du témoin non traité).

| Condition | Moyenne | Ecart type |
|---|---|---|
| Témoin non traité | 100 | 26 |
| Extrait selon l'invention à 0,1% (v/v) | 137 | 19 |

L'observation en microscopie confocale est présentée dans la figure 3.

### Conclusion :

Le collagène de type I est synthétisé en plus grande quantité lorsque la culture a été effectuée en présence de l'extrait d*'Hamamelis virginiana* à 0,1% (v/v) selon l'invention (Figure 3B) qu'en l'absence de l'extrait (Figure 3A). Ces résultats démontrent que l'extrait d*'Hamamelis virginiana* selon l'invention à 0,1% augmente la quantité et la qualité du réseau de fibres de collagène.

### Exemple 4 : Visualisation de l'inhibition de l'expression de l'élafine par un extrait selon l'invention:

### Protocole

L'effet de l'extrait d*'Hamamelis virginiana* obtenu à l'exemple 1a) a été testé à 0,1% en volume par rapport au volume total du milieu de culture sur un modèle de peau reconstruite de type Mimeskin™, connu de l'homme du métier. Brièvement, ce modèle de peau est obtenu par culture de fibroblastes humains normaux puis à partir du 28^{ème} jour de culture (J28), des kératinocytes humains normaux sont ajoutés et mis en prolifération jusqu'au 35^{ème} jour de culture (J35) puis leur différenciation est induite et l'ensemble des cellules est cultivé jusqu'au 56^{ème} jour (J56). Après 56 jours de culture, les cellules ont été rincées avec un tampon Phosphate Buffer Saline (PBS) contenant du calcium, du magnésium et des antibiotiques puis fixées en formaldéhyde durant 10 minutes. Trois conditions ont été évaluées :
- peau non irradiée et non traitée dite Témoin Négatif
- peau irradiée du 14 au 28^{ème} jour de culture (J14 à J28) et à partir du 42^{ème} jour (J42 à J56) par des UVA (longueur d'onde λ= 365nm) 1J/ cm² dite Témoin Irradié
- peau traitée à partir du 3^{ème} jour de culture par l'extrait d*'Hamamelis virginiana* obtenu à l'exemple 1a) à 0,1% dans le milieu de culture (J3 à J56) et irradiée du 14 au 28^{ème} jour de culture (J14 à J28) et à partir du 42^{ème} jour (J42 à J56) par des UVA (longueur d'onde λ= 365nm) 1J/ cm², dite Peau Traitée

Le modèle est paraffiné et des coupes de 5micromètres ont été effectuées.

Les coupes ainsi réalisées sont déparaffinées, mises sur lame et hydratées. Un démasquage enzymatique est effectué à la trypsine à 0,1% (v/v) pendant 10minutes à 37°C puis les lames sont saturées par une solution tampon de PBS-BSA 3% (albumine sérique bovine) pendant une heure. L'anticorps primaire anti-élafine de type polyclonal de lapin (Abcan™ ab-46774) est appliqué à la concentration de 5microgrammes par millilitre dans le milieu de culture PBS-BSA 1% pendant la durée de 16h à 4°C. Les lames sont lavées dans un tampon PBS pendant 5 minutes, ce lavage étant renouvelé deux fois. Un blocage des peroxydases endogènes est effectué avec une solution spécifique (solution DAKO™ DAB Real Blocking Perodixase pendant 30minutes en chambre humide à température ambiante). Les lames sont lavées dans un tampon PBS pendant 5 minutes, ce lavage étant renouvelé deux fois. L'anticorps secondaire (anti-lapin) couplé à la peroxydase est ajouté selon les recommandations du fournisseur et les lames sont lavées dans un tampon PBS pendant 5 minutes, ce lavage étant renouvelé deux fois. La révélation est effectuée par une solution spécifique (solution DAKO™ DAB chromogène dans une solution DAKO™).

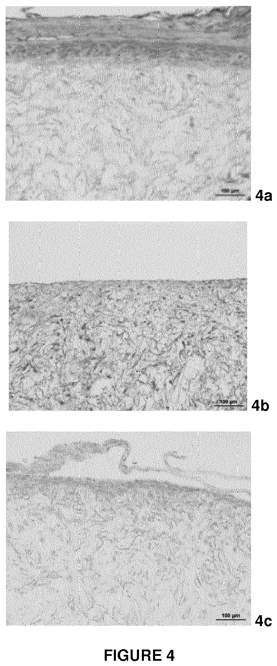

Enfin, les lames peuvent être observées au microscope confocal et une quantification de la surface de marquage est effectuée. Les résultats sont présentés dans le tableau 4 ci-après ainsi que dans la figure 4 a) pour le Témoin Négatif, 4b) pour le Témoin Irradié et 4c) pour la Peau Traitée.

### Résultats :

**Tableau 4 : valeur moyenne mesurée en pixels sur une aire prédéfinie au niveau du derme à l'aide d'un logiciel d'imagerie (ImaqeJ).**

| Condition | Moyenne en pixels |
|---|---|
| Témoin Négatif | 205000 |
| Témoin Irradié | 455000 |
| Peau Traitée | 65000 |

### Conclusion :

L'expression de l'élafine est induite par l'irradiation aux UVA (Figure 4b). Lorsque l'irradiation est effectuée en présence de l'extrait d*'Hamamelis virginiana* selon l'invention l'expression de l'élafine est diminuée (Figure 4c). Ceci démontre que l'extrait selon l'invention inhibe l'expression de l'élafine induite par les UVA.

### Exemple 5 : composition comprenant l'extrait selon la présente invention destinée à être incorporée dans une composition cosmétique (ingrédient cosmétique)

L'extrait d*'Hamamelis virginiana* est obtenu selon l'exemple 1a) et est mélangé avec les autres ingrédients de la formulation suivante :

| **Ingrédient** | **% en poids par rapport au poids total la composition** |
|---|---|
| Eau qsp | >50 % |
| extrait aqueux *d'Hamamelis virginiana* (Ex 1a) | 5% |
| Pentylène glycol | 1-5 % |
| Caprylyl glycol | 0,1-1 % |
| Gélifiant (gomme xanthane) | 0,1-1 % |

### Exemple 6 : compositions contenant l'extrait d'Hamamelis virginiana selon l'invention.

On procède selon les méthodes connues de l'homme de l'art pour mélanger ensemble les différentes parties A, B, C, D, E, ou F pour préparer une composition selon la présente invention.

Les « produits de l'invention » représentent un extrait d*'Hamamelis virginiana* et de préférence obtenu selon l'exemple 1a).

Les produits de l'invention peuvent également se présenter sous forme de liposomes contenant 5% de lécithine de soja et incorporant une solution de soja quaternisé (600 g en final) obtenus selon le mode de réalisation suivant :
30 g de lécithine de soja, 12 g de solution de soja quaternisé, 1,5 g d'extrait d*'Hamamelis virginiana* préparé selon l'exemple 1a) sont introduits dans un pilulier et dilué dans 447 g d'eau pure de laboratoire.

Après agitation magnétique pendant 10 minutes à température ambiante, le mélange est homogénéisé violemment pendant 10 minutes, en obtenant ainsi une solution liposomale dans laquelle les liposomes ont une dimension moyenne pouvant varier entre 100 et 800 nanomètres selon les conditions exactes de l'homogénéisation.

La suspension est ensuite laissée sous agitation douce pendant 1 heure. 90g de butylène glycol, 6g de phenoxyethanol et 6g d'hydroxyéthylcellulose (agent de gélification) sont ensuite ajoutés.

**Composition cosmétique 6a :**

| | | |
|---|---|---|
| A | Eau | qsp 100 |
| | Butylène glycol | 2 |
| | Glycérine | 3 |
| | Sodium Dihydroxycetyl Phosphate,Isopropyl Hydroxycetyl Ether | 2 |
| B | Glycol Stéarate SE | 14 |
| | Triisononaoin | 5 |
| | Octyl Cocoate | 6 |
| C | Butylène Glycol, Methylparaben, Ethylparaben, Propylparaben | 2 |
| | pH ajusté à 5,5 | |
| D | Produits de l'invention | 0,01 - 10 % |

**Composition cosmétique 6b :**

| | | |
|---|---|---|
| A | Eau | qsp 100 |
| | Butylène Glycol | 2 |
| | Glycérine | 3 |
| | Polyacrylamide, Isoparaffine, Laureth-7 | 2,8 |
| B | Butylène Glycol, Methylparaben, Ethylparaben, Propylparaben ; | 2 |
| | Phenoxyethanol, Methylparaben | , |
| | Propylparaben, Butylparaben, Ethylparaben | 2 |
| | Butylène Glycol | 0,5 |
| D | Produits de l'invention | 0,01 - 10 % |

**Composition cosmétique 6c :**

| | | |
|---|---|---|
| A | Carbomer | 0,50 |
| | Propylène Glycol | 3 |
| | Glycérol | 5 |
| | Eau | qsp 100 |
| B | Octyl Cocoate | 5 |
| | Bisabolol | 0,30 |
| | Dimethicone | 0,30 |
| C | Hydroxyde de Sodium | 1,60 |
| D | Phenoxyethanol, Methylparaben, Propylparaben, Butylparaben, Ethylparaben | 0,50 |
| E | Parfum | 0,30 |
| F | Produits de l'invention | 0,01 - 10 % |

**Composition dermatologique 6d sous forme d'une pommade**

| | | |
|---|---|---|
| A | Excipients | |
| | Polyéthylène base densité | 5,5 |
| | Paraffine liquid | qsp 100 |
| B | Produit de l'invention* | 0,001 -0,1 |

| | | |
|---|---|---|
| **L'extrait d'Hamamelis virginiana est celui décrit en exemple 1a) suivi d'une étape de stérilisation et de séchage.* | | |

## Revendications

1. Utilisation cosmétique non thérapeutique d'un extrait d*'Hamamelis virginiana* pour augmenter la formation des fibres de la matrice extracellulaire dans la peau et/ou les muqueuses et/ou du cuir chevelu pour augmenter la fermeté de la peau et/ou de muqueuses et/ou du cuir chevelu et/ou pour le soin et/ou le traitement des vergetures, l'extrait d*'Hamamelis virginiana* étant obtenu à partir des feuilles.

2. Utilisation selon la revendication 1 dans laquelle les fibres de la matrice extracellulaire sont les fibres élastiques et/ou les fibres de collagène.

3. Utilisation selon l'une quelconque des revendications 1 ou 2 pour diminuer la formation d'agrégats d'élastine.

4. Utilisation selon l'une quelconque des revendications 1 à 3 pour augmenter l'expression de LOXL, l'expression et/ou l'activité du collagène, l'expression et/ou l'activité de la fibuline 5, et/ou pour inhiber l'expression et/ou activité de l'élafine.

5. Utilisation selon l'une quelconque des revendications 1 à 4 dans laquelle l'extrait d*'Hamamelis virginiana* est obtenu par extraction aqueuse.

6. Utilisation selon l'une quelconque des revendications 1 à 5 dans laquelle l'extrait d*'Hamamelis virginiana* est obtenu par extraction à une température comprise entre 0°C et 30°C.

7. Utilisation selon l'une quelconque des revendications 1 à 6, dans laquelle l'extrait d*'Hamamelis virginiana* est dissout dans un solvant polaire choisi parmi l'eau, un alcool, un polyol, un glycol ou un de leurs mélanges.

8. Utilisation selon l'une quelconque des revendications 1 à 7 dans laquelle l'extrait *d'Hamamelis virginiana* est contenu dans une composition cosmétique contenant un excipient cosmétique topiquement acceptable.

9. Utilisation selon l'une quelconque des revendications 1 à 8 dans laquelle l'extrait d*'Hamamelis virginiana* est appliqué par voie topique sur la peau et/ou les muqueuses et/ou le cuir chevelu.

10. Utilisation selon l'une quelconque des revendications 8 ou 9 dans laquelle l'extrait d*'Hamamelis virginiana* est contenu à une concentration comprise entre 1.10⁻⁴ et 10% en poids, et avantageusement entre 1.10⁻⁴ et 5% et plus particulièrement entre 1.10⁻³ et 3% en poids par rapport au poids total de la composition cosmétique.

11. Utilisation selon l'une quelconque des revendications 8 à 10 dans laquelle la composition cosmétique contient moins de deux composés choisis parmi la cyclodextrine, le pentetate de pentasodium, l'acide phytique, le citrate de potassium, le citrate de sodium, le gluconate de potassium et le gluconate de sodium, avantageusement elle ne contient pas de composé choisi parmi la cyclodextrine, le pentetate de pentasodium, l'acide phytique, le citrate de potassium, le citrate de sodium, le gluconate de potassium et le gluconate de sodium.

12. Procédé de soin cosmétique **caractérisé en ce qu'**il comprend l'application par voie topique sur au moins une zone de peau et/ou des muqueuses et/ou du cuir chevelu d'un extrait d*'Hamamelis virginiana* pour augmenter la formation des fibres de la matrice extracellulaire dans la peau et/ou les muqueuses et/ou le cuir chevelu, préférentiellement les fibres élastiques et/ou les fibres de collagène, pour augmenter la fermeté de la peau et/ou de muqueuses et/ou du cuir chevelu et/ou pour le soin et/ou le traitement des vergetures, l'extrait d*'Hamamelis virginiana* étant obtenu à partir des feuilles.

13. Procédé de soin cosmétique selon la revendication 12 dans lequel l'extrait d*'Hamamelis virginiana* est tel que défini dans l'une des revendications 5 à 11.

14. Procédé de soin cosmétique selon l'une quelconque des revendications 12 ou 13 dans lequel la zone de peau et/ou muqueuse et/ou cuir chevelu est choisie parmi au moins le cuir chevelu, le cou, le décolleté, le ventre, les bras, les cuisses, les hanches, les fesses, la taille et/ou le visage, et préférentiellement le contour des yeux, les lèvres.

15. Extrait d*'Hamamelis virginiana* pour son utilisation par voie topique pour le soin et/ou le traitement dermatologique des pathologies impliquant une perte de fermeté et/ou d'élasticité de la peau et/ou des muqueuses et/ou du cuir chevelu choisies parmi les télangiectasies, l'élastose solaire et/ou la maladie cutix laxa, l'extrait d*'Hamamelis virginiana* étant obtenu à partir des feuilles.

16. Extrait *d'Hamamelis virginiana* pour utilisation selon la revendication 15 **caractérisé en ce qu'**il est tel que défini dans l'une des revendications 5 à 7.

17. Extrait d*'Hamamelis virginiana* pour utilisation selon l'une quelconque des revendications 15 ou 16, **caractérisé en ce qu'**il est présent dans une composition dermatologique comprenant un excipient dermatologique topiquement acceptable et **en ce qu'**il est contenu à une concentration finale comprise entre 1.10⁻⁴ et 10% en poids, et avantageusement entre 1.10⁻⁴ et 5% et plus particulièrement entre 1.10⁻³ et 3% en poids par rapport au poids total de la composition dermatologique.

18. Extrait d*'Hamamelis virginiana* pour utilisation selon la revendication 17, **caractérisé en ce que** la composition dermatologique contient moins de deux composés choisis parmi la cyclodextrine, le pentetate de pentasodium, l'acide phytique, le citrate de potassium, le citrate de sodium, le gluconate de potassium et le gluconate de sodium, avantageusement elle ne contient pas de composé choisi parmi la cyclodextrine, le pentetate de pentasodium, l'acide phytique, le citrate de potassium, le citrate de sodium, le gluconate de potassium et le gluconate de sodium.

## Patentansprüche

1. Nicht-therapeutische kosmetische Verwendung eines Extrakts von *Hamamelis virginiana* zur Erhöhung der Bildung von Fasern der extrazellulären Matrix in der Haut und/oder den Schleimhäuten und/oder der Kopfhaut zur Erhöhung der Straffheit der Haut und/oder der Schleimhäute und/oder der Kopfhaut und/oder zur Pflege und/oder zur Behandlung von Dehnungsstreifen, wobei der Extrakt von *Hamamelis virginiana* aus Blättern erhalten wird.

2. Verwendung gemäß Anspruch 1, wobei die Fasern der extrazellulären Matrix elastische Fasern und/oder Kollagenfasern sind.

3. Verwendung gemäß einem der Ansprüche 1 oder 2 zur Verringerung der Bildung von Elastinaggregaten.

4. Verwendung gemäß einem der Ansprüche 1 bis 3 zur Erhöhung der Expression von LOXL, der Expression und/oder der Aktivität von Kollagen, der Expression und/oder der Aktivität von Fibulin 5 und/oder zur Inhibierung der Expression und/oder der Aktivität von Elafin.

5. Verwendung gemäß einem der Ansprüche 1 bis 4, wobei der Extrakt von *Hamamelis virginiana* durch wässrige Extraktion erhalten wird.

6. Verwendung gemäß einem der Ansprüche 1 bis 5, wobei der Extrakt von *Hamamelis virginiana* durch Extraktion bei einer Temperatur zwischen 0°C und 30°C erhalten wird.

7. Verwendung gemäß einem der Ansprüche 1 bis 6, wobei der Extrakt von *Hamamelis virginiana* in einem polaren Lösungsmittel gelöst wird, ausgewählt aus Wasser, einem Alkohol, einem Polyol, einem Glykol und einer ihrer Mischungen.

8. Verwendung gemäß einem der Ansprüche 1 bis 7, wobei der Extrakt von *Hamamelis virginiana* in einer kosmetischen Zusammensetzung enthalten ist, die einen topisch annehmbaren kosmetischen Exzipienten enthält.

9. Verwendung gemäß einem der Ansprüche 1 bis 8, wobei der Extrakt von *Hamamelis virginiana* auf topischem Weg auf die Haut und/oder die Schleimhäute und/oder die Kopfhaut aufgebracht wird.

10. Verwendung gemäß einem der Ansprüche 8 oder 9, wobei der Extrakt von *Hamamelis virginiana* in einer Konzentration zwischen 1x10⁻⁴ und 10 Gew.-%, und vorteilhaft zwischen 1x10⁻⁴ und 5 Gew.-% und insbesondere zwischen 1x10⁻³ und 3 Gew.-%, bezogen auf das Gesamtgewicht der kosmetischen Zusammensetzung, enthalten ist.

11. Verwendung gemäß einem der Ansprüche 8 bis 10, wobei die kosmetische Zusammensetzung weniger als zwei Verbindungen enthält, ausgewählt aus Cyclodextrin, Pentanatriumpentetat, Phytinsäure, Kaliumcitrat, Natriumcitrat, Kaliumgluconat und Natriumgluconat, wobei sie vorteilhaft keine aus Cyclodextrin, Pentanatriumpentetat, Phytinsäure, Kaliumcitrat, Natriumcitrat, Kaliumgluconat und Natriumgluconat ausgewählte Verbindung enthält.

12. Verfahren zur kosmetischen Pflege, **dadurch gekennzeichnet, dass** dieses das Anwenden auf topischem Weg, auf mindestens eine Zone der Haut und/oder der Schleimhäute und/oder der Kopfhaut, eines Extrakts von *Hamamelis virginiana* zur Erhöhung der Bildung von Fasern der extrazellulären Matrix in der Haut und/oder den Schleimhäuten und/oder der Kopfhaut, vorzugsweise elastischen Fasern und/oder Kollagenfasern, zur Erhöhung der Straffheit der Haut und/oder der Schleimhäute und/oder der Kopfhaut und/oder zur Pflege und/oder zur Behandlung von Dehnungsstreifen, wobei der Extrakt von *Hamamelis virginiana* aus Blättern erhalten wird.

13. Verfahren zur kosmetischen Pflege gemäß Anspruch 12, wobei der Extrakt von *Hamamelis virginiana* wie in einem der Ansprüche 5 bis 11 definiert ist.

14. Verfahren zur kosmetischen Pflege gemäß einem der Ansprüche 12 oder 13, wobei die Zone der Haut und/oder der Schleimhaut und/oder der Kopfhaut mindestens ausgewählt ist aus der Kopfhaut, dem Hals, dem Dekolleté, dem Bauch, den Armen, den Schenkeln, den Hüften, dem Gesäß, der Taille und/oder dem Gesicht, und vorzugsweise der Kontur der Augen und der Lippen.

15. Extrakt von *Hamamelis virginiana* zu seiner Verwendung auf topischem Weg zur dermatologischen Pflege und/oder zur dermatologischen Behandlung von Pathologien, die einen Verlust der Straffheit und/oder der Elastizität der Haut und/oder der Schleimhäute und/oder der Kopfhaut implizieren, ausgewählt aus Telangiectasien, Sonnenelastose und/oder Cutix-laxa-Syndrom, wobei der Extrakt von *Hamamelis virginiana* aus Blättern erhalten wird.

16. Extrakt von *Hamamelis virginiana* zur Verwendung gemäß Anspruch 15, **dadurch gekennzeichnet, dass** dieser wie in einem der Ansprüche 5 bis 7 definiert ist.

17. Extrakt von *Hamamelis virginiana* zur Verwendung gemäß einem der Ansprüche 15 oder 16, **dadurch gekennzeichnet, dass** dieser in einer dermatologischen Zusammensetzung vorhanden ist, umfassend einen topisch annehmbaren dermatologischen Exzipienten, und dadurch, dass dieser bei einer Endkonzentration zwischen 1x10⁻⁴ und 10 Gew.-%, und vorteilhaft zwischen 1x10⁻⁴ und 5 Gew.-% und insbesondere zwischen 1x10⁻³ und 3 Gew.-%, bezogen auf das Gesamtgewicht der kosmetischen Zusammensetzung, enthalten ist.

18. Extrakt von *Hamamelis virginiana* zur Verwendung gemäß Anspruch 17, **dadurch gekennzeichnet, dass** die dermatologische Zusammensetzung weniger als zwei Verbindungen enthält, ausgewählt aus Cyclodextrin, Pentanatriumpentetat, Phytinsäure, Kaliumcitrat, Natriumcitrat, Kaliumgluconat und Natriumgluconat, wobei sie vorteilhaft keine aus Cyclodextrin, Pentanatriumpentetat, Phytinsäure, Kaliumcitrat, Natriumcitrat, Kaliumgluconat und Natriumgluconat ausgewählte Verbindung enthält.

## Claims

1. Non therapeutic cosmetic use of an extract of *Hamamelis virginiana* for increasing the formation of fibers of the extracellular matrix in the skin and/or mucous membranes and/or the scalp for increasing firmness of the skin and/or mucous membranes and/or the scalp and/or for the care and/or the treatment of stretch marks, the extract of *Hamamelis virginiana* being obtained from leaves.

2. Use according to claim 1 wherein the fibers of the extracellular matrix are elastic fibers and/or collagen fibers.

3. Use according to any of the claims 1 or 2 for diminishing the formation of elastin aggregates.

4. Use according to any of the claims 1 to 3 for increasing the expression of LOXL, the expression and/or the activity of collagen, the expression and/or the activity of fibulin 5, and/or for inhibiting the expression and/or the activity of elafin.

5. Use according to any of the claims 1 to 4 wherein the extract of *Hamamelis virginiana* is obtained by aqueous extraction.

6. Use according to any of the claims 1 to 5 wherein the extract of *Hamamelis virginiana* is obtained by extraction at a temperature between 0°C and 30°C.

7. Use according to any of the claims 1 to 6 wherein the extract of *Hamamelis virginiana* is dissolved in a polar solvent chosen from water, alcool, polyol, glycol or one of their mixtures.

8. Use according to any of the claims 1 to 7 wherein the extract of *Hamamelis virginiana* is contained in a cosmetic composition containing a topically acceptable cosmetic excipient.

9. Use according to any of the claims 1 to 8 wherein the extract of *Hamamelis virginiana* is applied by topical route onto the skin and/or the mucous membranes and/or the scalp.

10. Use according to any of the claims 8 or 9 wherein the extract of *Hamamelis virginiana* is present at a concentration comprised between 1.10⁻⁴ and 10% in weight, preferentially 1.10⁻⁴ and 5%, and more preferentially between 1.10⁻³ and 3% in weight relative to the total weight of the cosmetic composition.

11. Use according to any of the claims 8 to 10 wherein the cosmetic composition contains less than two compounds chosen from cyclodextrin, pentasodium pentetate, phytic acid, potassium citrate, sodium citrate, potassium gluconate and sodium gluconate, advantageously said composition does not contain a compound chosen from cyclodextrin, pentasodium pentetate, phytic acid, potassium citrate, sodium citrate, potassium gluconate and sodium gluconate.

12. Method of cosmetic care **characterized by** comprising the application by topical route onto at least one area of the skin and/or mucous membranes and/or the scalp of an extract of *Hamamelis virginiana* for increasing the formation of fibers of the extracellular matrix in the skin and/or mucous membranes and/or the scalp, preferentially elastic and/or collagen fibers, for increasing firmness of the skin and/or mucous membranes and/or the scalp and/or for the care and/or the treatment of stretch marks, the extract of *Hamamelis virginiana* being obtained from leaves.

13. Method of cosmetic care according to claim 12 wherein the extract of *Hamamelis virginiana* is as defined in any one of the claims 5 to 11.

14. Method of cosmetic care according to any of the claims 12 or 13 wherein the area of the skin and/or the mucous membrane and/or the scalp is chosen among at least the scalp, the neck, the neckline, the belly, the arms, the thighs, the hips, the buttocks, the waist and/or the face, and preferentially the eyes contour, the lips.

15. Extract of *Hamamelis virginiana* for its use by topical route for the dermatological care and/or the treatment of pathologies involving a loss of firmness and/or elasticity of the skin and/or mucous membranes and/or the scalp chosen from telangiectasias, solar elastosis and/or cutix laxa, , the extract of *Hamamelis virginiana* being obtained from leaves.

16. Extract of *Hamamelis virginiana* for it use according to claim 15 **characterized in that** said extract is as defined in any of the claims 5 to 7.

17. Extract of *Hamamelis virginiana* for its use according to any of the claims 15 or 16, **characterized in that** said extract is present in a dermatological composition comprising a topically acceptable dermatologic excipient and wherein said extract is contained in a final concentration comprised between 1.10⁻⁴ and 10% in weight, and advantageously between 1.10⁻⁴ and 5% in weight and more particularly between 1.10⁻³ and 3% in weight relative to the total weight of the dermatological composition.

18. Extract of *Hamamelis virginiana* for its use according to claim 17, **characterized in that** the dermatological composition contains less than two compounds chosen from cyclodextrin, pentasodium pentetate, phytic acid, potassium citrate, sodium citrate, potassium gluconate and sodium gluconate, advantageously said composition does not contain a compound chosen from cyclodextrin, pentasodium pentetate, phytic acid, potassium citrate, sodium citrate, potassium gluconate and sodium gluconate.


### SEQUENCE LISTING

<110> BASF BEAUTY CARE SOLUTIONS FRANCE SAS
   ANDRE, Valérie
   GAILLARD, Christelle
   CENIZO, Valérie
<120> UTILISATION COSMETIQUE ET/OU DERMATOLOGIQUE D'UN EXTRAIT D'HAMAMELIS VIRGINIANA
<130> 1H197810 0087 FR BN
<160> 4
<170> PatentIn version 3.3
<210> 1
   <211> 20
   <212> DNA
   <213> Artificial
<220>
   <223> Amorce sens actine
<400> 1
   gtggggcgcc ccaggcacca 20
<210> 2
   <211> 24
   <212> DNA
   <213> Artificial
<220>
   <223> Amorce anti-sens actine
<400> 2
   ctccttattg tcacgcacga tttc 24
<210> 3
   <211> 19
   <212> DNA
   <213> Artificial
<220>
   <223> Amorce sens LOXL
<400> 3
   gacttcggca acctcaagc 19
<210> 4
   <211> 20
   <212> DNA
   <213> Artificial
<220>
   <223> Amorce anti-sens LOXL
<400> 4
   tgttgcagaa acgtagcgac 20